# EUROPEAN PATENT APPLICATION

(11) **EP 1 005 877 A2**
(43) Date of publication of application: **07.06.2000**
(21) Application number: 99308997.8
(22) Date of filing: 11.11.1999
(51) Int. Cl.: A61M 16/04

(54) **Cuffed tubes**

(30) Priority: 03.12.1998 GB 9826428; 02.03.1999 GB 9904757
(71) Applicant: Smiths Industries Public Limited Company, London, NW11 8DS (GB)
(72) Inventor: Pagan, Eric, Hythe, Kent CT21 6DN (GB)
(74) Representative: Flint, Jonathan McNeill

(57) **Abstract**

A cuffed tracheal tube has a cuff 10, 30 that tapers along its length from a diameter at its patient end less than that of the trachea to a diameter at its machine end greater than that of the trachea. The cuff 10, 30 may have two annular channels 31 and 32 formed around it and spaced from one another, the region 35 between the channels being approximately matched in diameter to that of the trachea

## Description

This invention relates to cuffed medico-surgical tubes of the kind for insertion in an elongate body cavity, the tube having a tubular shaft and a cuff towards its patient end that can be expanded to seal with the cavity without stretching the cuff.

Various medico-surgical tubes, such as tracheal tubes, have a cuff encompassing the tube, which can be inflated to seal with the body cavity in which the tube is inserted. The seal serves several functions. It retains the tube in the correct position, it enables positive gas pressure to be applied to the patient and prevents the escape of anaesthetic gases, and it also helps prevent secretions formed in the trachea entering the lower airway. The cuffs take various different forms. Usually, they have a cylindrical or rounded shape, or they may have a pear shape, such as in the tube sold under the Profile trade mark by SIMS Portex Limited. It has been proposed previously that a cylindrical cuff be moulded with a series of annular corrugations, as described in US3810474, to form a labyrinth seal between the trachea and the tube. GB 2326099 describes a cuff with annular channels formed by reinforcement bands and having a tapering shape, reducing in diameter from the patient end. The precise shape of cuff on a tracheal tube has been found to be critical in producing a seal that is effective in preventing leakage of secretions past the cuff. These secretions are produced in the trachea above the cuff and collect on the cuff during use. The positive/negative cyclical pressure variations during patient ventilation can cause axial displacement of the tube shaft relative to the cuff and apply a peristaltic pumping effect to the cuff. It is important to keep to a minimum the leakage of secretions past the cuff into the patient's respiratory passages because they are believed to be a major cause of pneumonia-type infections following ventilation.

Some cuffs are designed to operate at low pressure with a high volume but these can allow folds to form in the cuff material, providing tracks along which secretions can flow. The best form of seal is produced by an optimally sized cuff producing a seal around its circumference unbroken by any fold of the material. It is difficult, however, to provide such a cuff of optimal size because of the wide variation in tracheal shapes and sizes.

It is an object of the present invention to provide a cuffed tube.

According to the present invention there is provided a cuffed medico-surgical tube of the above-specified kind, characterised in that the cuff has a contact region that in its natural, unstretched state increases along its length from a first diameter less than that of the cavity to a second diameter greater than that of the cavity.

The patient end of the cuff preferably has a smaller diameter than the machine end of the cuff. The cuff may have steeply tapering regions extending from the wall of the shaft to respective ends of the contact region. The contact region is preferably inclined in its natural state relative to the axis of the tube with a gradient of between about 4:1, such as for adult sizes, to about 3:1, such as for paediatric sizes. The wall thickness of the cuff may be between about 60 and 90 micron. The contact region preferably has a diameter of about 17mm at one end and a diameter of about 30mm at its opposite end. The cuff may have at least one annular channel extending around the contact region and preferably has two annular channels spaced from one another along the length of the region to divide the contact region into three parts. The part of the contact region between the two channels preferably has a diameter approximately equal to the diameter of the body cavity.

A cuffed endotracheal tube according to the present invention, will now be described, by way of example, with reference to the accompanying drawings, in which:
- Figure 1: is a side elevation view of the tube;
- Figure 2: is an enlarged side elevation view showing the cuff to a greater scale; and
- Figure 3: is an enlarged side elevation view of an alternative cuff

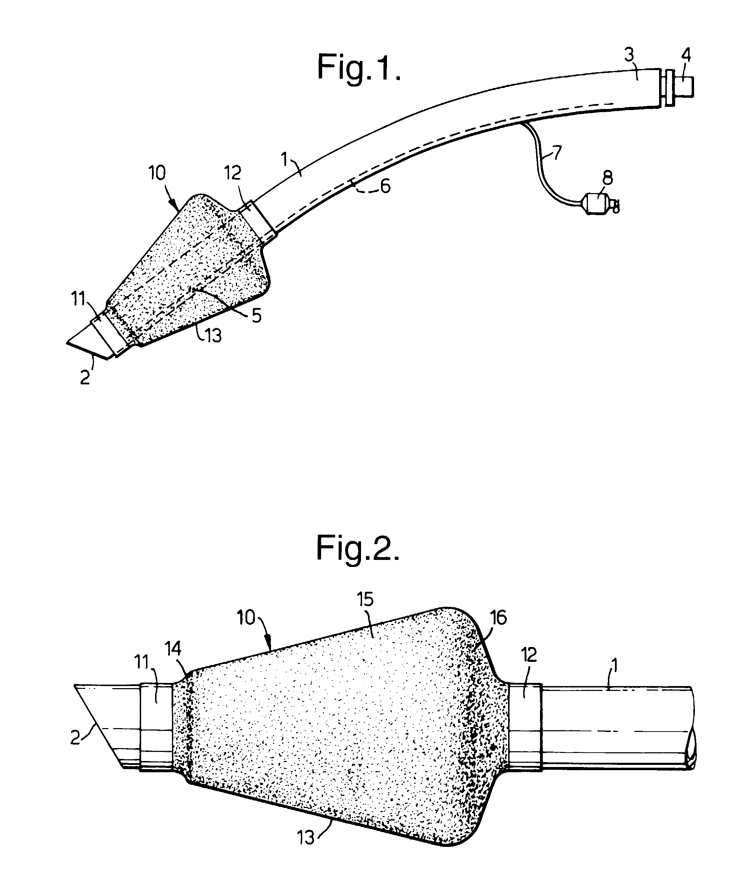

With reference first to Figures 1 and 2, the tube has an extruded shaft 1 of a bendable plastics such as PVC, the tube being curved along its length in the usual way to follow the anatomy of the patient. At its patient end 2, the tube is open and bevelled; at its machine end 3, the tube is cut square and has a connector 4 inserted within it by which the tube is connected to a patient ventilator or anaesthetic circuit, not shown.

Just rearwardly of the patient end 2 an inflatable cuff 10 is attached to the outside of the tube by means of opposite end collars 11 and 12, the cuff being adapted to seal with an elongate body cavity in the form of the patient's trachea. The central, inflatable part 13 of the cuff is located over an opening 5 through the outer wall of the shaft into a minor, inflation lumen 6 extruded within the wall of the shaft and extending along its length. The lumen 6 is closed at the patient and machine ends of the tube and connects with one end of a small bore inflation line 7 towards the machine end of the tube. A combined connector and inflation indicator 8 of a conventional kind is connected at the opposite end of the inflation line 7.

The central, inflatable part 13 of the cuff 10 has a novel shape provided by three regions 14, 15 and 16. The forward, patient end region 14 tapers rearwardly at a relatively steep angle to the forward end of the central, contact region 15 where it has a diameter of about 17mm, in a tube for use with a typical adult patient. The dimensions and shape of the cuff 10 are described when the cuff is inflated to the ISO Standard Cuff Resting Diameter pressure of 30mm H₂O, in its natural state, that is, unconstrained outside the trachea. The cuff is substantially inflated during use to a pressure insufficient to stretch the material from its natural shape. The contact region 15 extends for about 26mm rearwardly and tapers, at a less steep angle than the forward region 14, to an increased diameter at its rear end of about 30mm. The rear end region 16 tapers rearwardly, inwardly at a steep angle, to the rear end collar 12. The overall length of the cuff 10 is about 35mm including the collars 11 and 12. The central, contact region 15 has a gradient of about 4:1 relative to the axis of the tube, the contact region increasing smoothly in diameter along its length from the diameter at its forward end of 17 mm, which is less than that of the smallest adult trachea, to the diameter of 30 mm at its rear end, which is greater than that of the largest adult trachea. For smaller diameter tubes, such as those used in paediatric patients, the minimum diameter of the cuff in the contact region would be about 5 mm and the maximum diameter would be typically about 20mm, also the gradient may be steeper, such as about 3:1. The cuff is made by blow moulding from a polymeric plasticized PVC, such as Teknor Apex, and preferably has a wall thickness of about 6 micron, although this may be slightly greater, such as up to about 9 micron.

The taper on the contact region 15 ensures that, at some point along its length, the diameter of the cuff 10 is exactly matched with that of the trachea, providing an unbroken annular seal around the cuff. The maximum diameter of the cuff 10 is preferably not such as to enable significant folding of the cuff material in the larger part of the cuff. It has been found that the cuff of the present invention provides a better seal against flow of secretions than previous cuffs.

The end regions 14 and 16, which taper away from the contact region 15, stabilize the cuff 10, when inflated, against axial displacement relative to the tube. In some cuffs, however, the forward end regions may be omitted, the contact region abutting directly the patient end collar and the rear end region may be a simple vertical wall extending to the machine end cuff.

There are various modifications that could be made to the invention. The cuff could be reversed so that the smaller end of the contact region is located towards the machine end and the larger end is located towards the patient end. The rear end collar on the cuff could be turned inside out and attached to the tube within the inflatable part of the cuff so as to enable a suction lumen to remove secretions right up to the inflated part, as described in GB2250440. The contact region could form a part of a longer cuff extending above the vocal folds of the patient. The cuff of the present invention could be used in combination with other cuffs. The cuff could be expanded in other ways than by inflation air, such as, for example, by means of a resilient material within the cuff so that it has a naturally expanded state and is reduced in size for insertion and removal by applying a negative, vacuum pressure.

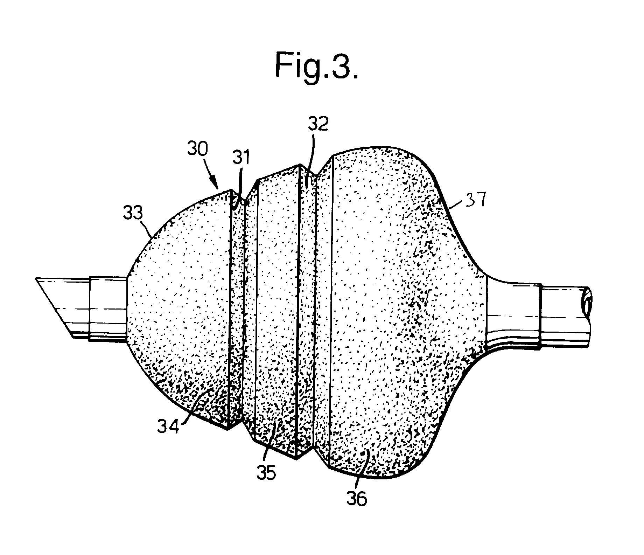

As shown in Figure 3, an alternative cuff 30 has two annular channels 31 and 32 formed around its exterior surface and spaced from one another along the length of the cuff. Different numbers of channels could be used. These channels divide the cuff 30 into the following parts or regions: a patient-end tapering region 33; a patient-end contact region 34; the first, patient-end channel 31; an intermediate contact region 35; the second, machine-end channel 32; a machine-end contact region 36; and a machine-end tapering region 37. The cuff 30 tapers externally along its central region provided by the three contact regions 34, 35 and 36, having a smaller diameter at its patient end compared with its machine end. The cuff size is selected so that the diameter of the central contact region 35 is matched as closely as possible to the expected diameter of the patient's trachea.

The channels 31 and 32 have a V-shape section but they could have any other shape, such as with a flat floor and square side walls, or walls that taper inwardly or outwardly.

The channels 31 and 32 may be produced by moulding into the cuff 30 or by attaching two reinforcing bands around the cuff so as to prevent these reinforced regions expanding outwardly to the same extent as the adjacent regions on either side.

The channels 31 and 32 divide the cuff 30 into three contact regions 34 to 36 of different diameters. Preferably the central region 35 is of the correct size but, even if the cuff 30 were not exactly matched to the trachea, one of the other regions should be of the correct size to form an effective seal, the other two regions being either too large or too small. If a region is too large, the cuff may crease in that region, allowing multiple small passages for the seepage of a relatively small volume of secretions; if it is too small, there will be a gap around the region allowing relatively unrestricted flow of secretions. Providing the largest diameter region towards the patient end ensures that the contact region 36 at the machine end either forms a creased seal or an effective seal. If even the largest diameter region 36 were too small, the cuff would not prevent leakage of gas and this would be apparent to the anaesthetist. It is believed that secretions are more effectively prevented from passing the cuff 30 in the arrangement of the present invention than if the cuff were to taper in the opposite direction, since there would be a risk, in such an arrangement, of a small diameter contact region at the machine end allowing relatively unimpeded flow of secretions to an adjacent region of the correct size. Whilst the contact region of the correct diameter is able to block the majority of secretions when secretions flowing up to this region are at a low level, as reduced by the larger diameter region, there would be a greater seepage past the correct diameter contact region if this flow were not reduced to such an extent.

The cuff of Figure 3 appears to maintain an effective seal with the trachea when the patient is moved. It is often necessary during, or following surgery to move a patient between a supine position to one where he lies on one side. Such movement often leads to a small rotational displacement of the tracheal tube about its axis. With the tube of the present invention, it is thought that any twisting force applied to the cuff 30 would be accommodated mainly in the machine end contact region 36 and that the oversize nature of this region means that the effectiveness of the seal in this region will not be substantially affected. The channel 32 helps isolate any twisting action in the machine end contact region 36 from the adjacent sealing region 35.

The invention is not restricted to tracheal tubes but could be used in other cuffed medico-surgical tubes for insertion in another elongate body cavity where the diameter of the cuff tapers along its length from a diameter less than that of the cavity to a diameter greater than that of the cavity.

## Claims

1. A cuffed medico-surgical tube for insertion in an elongate body cavity, the tube having a tubular shaft (1) and a cuff (10, 30) towards its patient end that can be expanded to seal with the cavity without stretching the cuff, characterised in that the cuff (10, 30) has a contact region (15, 34, 35, 36) that in its natural, unstretched state increases along its length from a first diameter less than that of the cavity to a second diameter greater than that of the cavity.

2. A tube according to Claim 1, characterised in that the patient end of the cuff (10, 30) has a smaller diameter than the machine end of the cuff.

3. A tube according to Claim 1 or 2, characterised in that the cuff (10, 30) has steeply tapering end regions (14, 16, 33, 37) extending from the wall of the shaft to respective ends of the contact region (15, 34, 35, 36).

4. A tube according to any one of the preceding claims, characterised in that the contact region (15, 34, 35, 36) is inclined in its natural state with a gradient of between about 4:1 to about 3:1 relative to the axis of the tube.

5. A tracheal tube according to Claim 4, characterised in that the gradient of the contact region (15, 34, 35, 36) is about 4:1 for adult sizes and about 3:1 for paediatric sizes.

6. A tube according to any one of the preceding claims, characterised in that the wall thickness of the cuff (10, 30) is between about 60 and 90 micron.

7. A tracheal tube according to any one of the preceding claims, characterised in that the contact region (15, 34, 35, 36) has a diameter of about 17mm at one end and a diameter of about 30mm at its opposite end.

8. A tube according to any one of the preceding claims, characterised in that the cuff (30) has at least one annular channel (31, 32) extending around the contact region (34, 35, 36).

9. A tube according to Claim 8, characterised in that the cuff has two annular channels (31 and 32) extending around the contact region spaced from one another along the length of the region to divide the contact region into three parts (34, 35 and 36).

10. A tube according to Claim 9, characterised in that the part (35) of the contact region between the two channels (31 and 32) has a diameter approximately equal to the diameter of the body cavity.
